Europäisches Patentamt

(19) European Patent Office

Office européen des brevets

(11) Publication number: **0 030 782**

**B1**

## (12) EUROPEAN PATENT SPECIFICATION

(45) Date of publication of patent specification: **18.01.84**

(51) Int. Cl.³: **C 01 G 55/00, A 61 K 33/24**

(21) Application number: **80302802.6**

(22) Date of filing: **14.08.80**

(54) A process of preparing platinum complexes.

(30) Priority: **16.08.79 US 65553**

(43) Date of publication of application:
**24.06.81 Bulletin 81/25**

(45) Publication of the grant of the patent:
**18.01.84 Bulletin 84/3**

(84) Designated Contracting States:
**AT BE CH DE FR GB IT LI LU NL SE**

(56) References cited:
**US - A - 4 075 307**
**US - A - 4 079 121**

**CHEMICAL ABSTRACTS, vol. 86, no. 24, June 13. 1977, ref. 182241k page 742 Columbus, Ohio, US C. LOCK et al.: "The structure of platinum anticancer agents and their metabolites"**

(73) Proprietor: **MPD Technology Corporation**
**681 Lawlins Road**
**Wyckoff New Jersey 07481 (US)**

(72) Inventor: **Rhoda, Richard Noble**
**18 Somerset Drive**
**Suffern New York 10901 (US)**
Inventor: **Crosby, Jeffrey Norman**
**18 Ludlow Road**
**Kidderminster Worcester Dylo INW (GB)**

(74) Representative: **Hedley, Nicholas James Matthew et al,**
**Thames House (fifth floor) Millbank**
**London SWIP 4QF (GB)**

# 0 030 782

## A process of preparing platinum complexes

The present invention relates to the preparation of cis-diamminedichloroplatinum(II) and of intermediate compounds used in such preparation.

Cis-diamminedichloroplatinum(II) is not a new compound. It was first reported in 1844 and named Peyrone's chloride after its inventor. Recently, this compound has been of particular interest because it has been found to have anti-cancer activity. When used as an anti-cancer agent the compound must be exceedingly pure. Specifications set up by the National Cancer Institute (NCI) require that to be acceptable the salt should contain no more than 1% of the trans-isomer. There is also evidence that the presence of any ionic species, e.g. Magnus' green salt and silver, in the final product should be as low as possible. Because platinum is so expensive, the yields should be high, otherwise the process would not be commercially acceptable. Several syntheses are known but all give unacceptable quantities of both the trans-isomer and the double Magnus' green salt, $[Pt(MH_3)_4][PtCl_4]$ as co-precipitates. A few companies are presently preparing cis-Pt(II) salts of acceptable purity but the methods used are proprietary and are kept secret.

R. J. Speer et al in "Cancer Chemotherapy Reports" Part I *59* (3), May/June 1975 indicate the cis-diamminedichloroplatinum(II) is rather toxic and that it has a low therapeutic index that is very dependent on its purity. They also recommend alternative routes to the synthesis and purification of the compound. One method for synthesis is that suggested in the literature by Kauffman and Cowan (Inorg. Syn. *7* 239 (1963)) and another by Dhara (Indian J. Chem. *8* 193 (1970). The method suggested by Dhara was carried out on such small scale that the presence of an unacceptable amount of Magnus' green salt would scarcely have been visible. When we attempted to follow the synthesis on a larger scale, we obtained an unacceptable amount of Magnus' green salt and found that the yield was by no means greater than 95% as reported. The purification techniques recommended are also rather sparse in details, the details are lacking in conditions used in the various steps in the alternative methods of synthesis. The present method is an improvement over the known methods with respect to specific steps and with respect to the overall method for producing cis-$[Pt(MH_3)_2Cl_2]$.

In accordance with the present invention there is provided a process which comprises a step in which an aqueous solution of ammonium hydroxide is added to an aqueous dispersion of $M_2PtI_4$ (where M represents sodium of potassium) to give a cis-$[Pt(NH_3)_2I_2]$ complex in such a manner that the pH does not exceed 7.5. Preferably the pH does not exceed 7. In order to minimise any local rise in pH during the actual process of adding $MH_4OH$, the addition of $NH_4OH$ is preferably controlled by slow addition, stirring the mixture vigorously during the addition, and by monitoring the pH during the addition. Under these conditions, yields upwards of about 91% cis-$Pt(NH_3)I_2$ can be obtained, and typically yields of 91—97%. Using this technique as a step in a route in preparing cis-$Pt(NH_3)_2Cl_2$ as the product, the crude product can be obtained with little or no Magnus' green salt and less than 1% of the transisomer.

The temperature at which the process is performed is important. At temperatures below 40°C the reaction proceeds slowly and at high temperatures, e.g. above 60°C, ammonia boils off. It has been found that a satisfactory way of performing the reaction is to increase the temperature as the reaction proceeds and preferably increasing the temperature step-wise within the range of from 40°C to 60°C.

As a further aspect of the invention cis-diamminediaquaplatinum(II) nitrate (i.e. cis-$[Pt(NH_3)_2(H_2O)_2](NO_3)_2$) is prepared from the cis-diamminediiodoplatinum(II) salt thus obtained by treating the cis-$[Pt(NH_3)_2I_2]$ with an aqueous solution of $AgNO_3$, preferably by a method comprising slowly adding the cis-diamminediiodoplatinum(II) to an aqueous solution of silver nitrate at room temperature and with rapid stirring and then warming the solution to no higher than about 50°C. The convenrsion for this method can be essentially quantitative.

To produce the cis-diaqua salt with a minimum concentration of silver ions in solution, the amount of $AgNO_3$ used should be monitored carefully. For example, the $AgNO_3$ may be used slightly under stoichiometric amount. After separation of the silver iodide precipitate, a small amount (e.g., 0.5 gram relative to 200 g of product in 2500 ml solution) of solid KCl (or NaCl) may be stirred into the filtrate to precipitate residual $Ag^+$ and the resultant product filtered to remove any precipitate. Alternatively, unreacted $Ag^+$ in solution can be avoided by monitoring the $Ag^+$ content using a Ag/AgCl electrode.

In still another aspect of the present invention cis-diamminedichloroplatinum(II) may be produced in high purity and in high yield from the cis-diamminediaquaplatinum(II) nitrate thus obtained by a method comprising rapidly adding MCl (M=, e.g. K, Na) to a solution of cis-diamminediaqua-platinum(II) nitrate at room temperature, warming the mixture to a temperature of from 65°C to 75°C, e.g., 70°C, and holding it at that temperature to allow completion of the reaction, permitting the resultant product to cool to a temperature, advantageously to no lower than 10°C, e.g. to about 15°C and preferably to room temperature, and then without permitting the product to stand, separating the precipitate from the solution.

By using the combined preparation steps given above for preparing the cis-diamminedichloro- platinum(II) from the tetraiodoplatinum(II) salt high yields of the crude product can be obtained with substantially no Magnus' green salt and less than 1% trans-isomer.

The crude cis-diamminedichloroplatinum(II) can be purified by one or more recrystallizations from dilute HCl (about 0.1 M) by adding the cis-diamminedichloroplatinum(II) to the dilute hydrochloric acid solution, heating to 100°C, filtering hot and permitting the solution to cool to room temperature and/or by recrystallization at room temperature from N,N'-dimethylformamide (DMF) with 0.1 N HCl addition.

In accordance with another aspect of the present invention the tetraiodo salt of platinum(II) may be obtained from $M_2PtCl_4$ (where M represents sodium or potassium), Preferably by admixing an aqueous solution of KI and an aqueous solution of $M_2PtCl_4$ at a temperature not in excess of 85°C. $M_2PtCl_4$ may be obtained from $M_2PtCl_6$, preferably by reducing it with hydrazine dihydrochloride by mixing aqueous solutions of the reactants at substantially room temperature.

To ensure high purity of the end product, the synthesis should preferably be made with very high purity elemental platinum as the initial material and very high purity reagents. Highly pure chloroplatinic acid is commercially available. If desired, elemental platinum can be converted to chloroplatinic acid, e.g. with aqua regia, using methods known in the art.

Chloroplatinic acid can be converted to the insoluble $M_2PtCl_6$ by treatment with potassium or sodium chloride. This reaction proceeds in an aqueous medium typically at a temperature in the range of 55° to 60°C, preferably about 55°C. When cooled to 0°C a very good yield of the platinum salt can be obtained. No alcohol is necessary.

Although the process of the present invention can be carried out using sodium or potassium platinum complexes, it is preferred to use the potassium platinum complexes since they give rise to better yields than the corresponding sodium complexes.

A process in accordance with the present invention will now be described by way of example only:

Example 1

It will be appreciated that tetrachloro and hexachloro salts of platinum(II) and (IV) such as $K_2PtCl_4$ and $K_2PtCl_6$ are commercially available and that the synthesis of cis-[Pt(NH$_3$)$_2$Cl$_2$] need not be started from $H_2PtCl_6$, but can be started at any stage in the synthesis using commercially available chemicals. The following description of the process, however, will describe a preferred embodiment of the present invention starting with high purity chloroplatinic acid to synthesize material which will meet the American National Cancer Institute (NCI) specification on December 9, 1975 for compound No. NSC-119,875 (cis-Pt[NH$_3$]$_2$Cl$_2$). In the following description and the examples which follow all reactions are carried out using a magnetic stirrer, all materials used are very pure, and room temperature means about 25°C. Yields were determined by wet chemical analysis initially and thereafter gravimetrically, except if otherwise indicated.

Step 1 (state of the art)
Reaction: $H_2PtCl_6 + 2KCl \rightarrow K_2PtCl_6 + 2HCl$

An aqueous solution containing roughly 0.25 moles per litre of chloroplatinic acid heated to about 55°C±5°C is treated by slow, dropwise addition of a concentrated aqueous solution of KCl. The amount of KCl used is stoichiometric for complete conversion to $K_2PtCl_6$ which forms as a yellow precipitate. The reaction mixture need not be heated. However, it is preferred to elevate the temperature moderately, e.g. to about 50°C, to ensure that the reaction goes to completion and that no KCl gets entrapped in the $K_2PtCl_6$. Also, to ensure complete conversion a very slight excess of KCl can be added. The product is cooled to 0°C and permitted to stand, e.g. overnight, to ensure complete precipitation.

After filtration, the residue is washed with cold distilled water, e.g., with three 500 ml portions of water, and then with absolute ethyl alcohol, e.g., with a 500 ml portion of alcohol. The washed residue is dried, e.g., for 2 hours at 110°C. The filtrate is kept for platinum recovery. A yield of 95% has been obtained.

Step 2 (state of the art)
Reaction: $2K_2PtCl_6 + N_2H_4 \cdot 2HCl \rightarrow 2K_2PtCl_4 + 6HCl + N_2$

The dried $K_2PtCl_6$ (about 0.5 mole) is dispersed in 2000 ml of distilled water and an aqueous solution of $N_2H_4 \cdot 2HCl$ containing a 5% excess of stoichiometric amount, is added dropwise with vigorous stirring. The addition is done at room temperature and the flow of reagent is adjusted so that addition takes 30 minutes. Thereafter the resultant mixture is warmed to 50°—60°C until the yellow precipitate disappears. This takes typically one hour. Thereafter the resultant red solution is heated to 85°C and held there for 15 minutes, then cooled to room temperature and refrigerated overnight. Essentially complete conversion to the tetrachloride can be achieved without decomposition to elemental Pt.

To ensure the removal of any unreacted $K_2PtCl_6$, the product of Step 2 is cooled to about 0°C and permitted to stand. For convenience the product may be refrigerated overnight. In carrying out Step 2 care should be taken to add sufficient hydrazine to react with the $K_2PtCl_6$, only slight excess, e.g. 5% is

3

used, since too great an excess of hydrazine causes reduction to Pt°. The vessel used for the reaction should be free from cracks or scratches which may serve as sites for nucleation of Pt°.

Step 3 (state of the art)
Reaction: $K_2PtCl_4+4KI \rightarrow K_2PtI_4+4HCl$
The potassium tetrachloroplatinum(II) solution is warmed to 40°C and an aqueous solution of 10 molar KI is added dropwise in sufficient amount for complete conversion to the tetraiodo salt. A black precipitate of $K_2PtI_4$ begins to form. Typically this takes about 1 hour, after which the solution is heated to 85°C and held there for 15 minutes. Essentially complete conversion can be achieved. The product is cooled to at least 40°C for use in the next step.

Step 4
Reaction: $K_2PtI_4+2NH_4OH \rightarrow cis\text{-}Pt(NH_3)_2I_2+2KI+2H_2O$
To the $K_2PtI_4$ containing dispersion of Step 3 at 40°C, a diluted solution of $NH_4OH$ is added dropwise using a pH meter so that pH 7 is not exceeded during the addition. $K_2PtI_4$ has a pH of about 4.9 in water at room temperature. However, sufficient $NH_4OH$ must be added to neutralize the HCl in solution as well as to react with the $K_2PtI_4$. (If the $K_2PtI_4$ is in a pure water dispersion the reaction would only require sufficient $NH_4OH$ to react with the $K_2PtI_4$, using proper precautions with regard to pH, temperature and the manner in which the $NH_4OH$ is added.) The dilute $NH_4OH$ is added until the black precipitate disappears, typically 3 to 4 hours. The solution is warmed to 60°C for 1 hour, adding dilute $NH_4OH$ as needed to pH=7, and then the reaction mixture is cooled to room temperature. A dark greenish brown precipitate forms which is filtered. The resultant precipitate is washed with distilled water and then absolute ethyl alcohol, e.g., three washes with 500 ml water and one with 500 ml alcohol. The filtrate is kept for platinum recovery. The yield is typically about 91% for Steps 2, 3 and 4, but a yield as high as 97% has been obtained.

Step 5
Reaction: $cis\text{-}Pt(NH_3)_2I_2+2AgNO_3+2H_2O \rightarrow cis\text{-}[Pt(NH_3)_2(H_2O)_2](NO_3)_2+2AgI$
After dissolving $AgNO_3$ in distilled water, solid $cis\text{-}Pt(NH_3)_2I_2$ is added slowly to the solution at room temperature with rapid stirring. Typically addition of the cis-diamminediiodoplatinum(II) to the solution of $AgNO_3$ takes about 30 minutes, and the resultant mixture is stirred for an additional period of time, e.g. about 10 minutes, and then it is warmed to 50°C and held there for a short period of time, e.g., 15 minutes, to ensure complete reaction. Thereafter the reaction mixture is cooled to room temperature and filtered to remove the AgI precipitate.
The AgI precipitate is washed, e.g., with three 500 ml portions of hot distilled water.
When it is particularly desirable to minimize the silver level in the product, the $AgNO_3$ reagent is used slightly below the stoichiometric amount. To the separated filtrate after reaction with $AgNO_3$, a small amount of solid KCl, e.g. 0.5 g KCl, is added and after stirring at room temperature for a short period, the product is filtered. Filtration can be carried out through the AgI precipitate and the residue can be kept for recovery of Ag.
The yield of the cis-diaqua salt in solution is quantitative. The high yields are ensured by adding the cis-diamminediiodoplatinum(II) to the $AgNO_3$ solution. At room temperature the KCl serves to precipitate $Ag^+$ in solution. When the $AgNO_3$ is added, for example, to a hot solution of the iodo salt, as recommended in the art, the yields are not as high. In addition if reaction temperature is permitted to go above 50°C, e.g. to 65°C, then yields are not as high.

Step 6
Reaction: $cis\text{-}[Pt(NH_3)_2(H_2O)_2](NO_3)_2+2KCl \rightarrow cis\text{-}Pt(NH_3)_2Cl_2+2KNO_3+2H_2O$
To the filtrate of Step 5 at room temperature and with stirring is added solid KCl in an amount of 10% excess, as quickly as possible. The mixture is warmed to 70°C and held there for completion of the reaction, typically 1 hour, and the product allowed to cool to room temperature while stirring to avoid temperature gradient. As soon as the product is at room temperature, it is filtered and the residue washed. Washing is carried out with three 500 ml portions of distilled water followed by one 500 ml portion of absolute ethyl alcohol.
Co-precipitation of impurities can be avoided by cooling only to room temperature. Further cooling below room temperature, e.g., to 0°C resulted in the appearance of traces of Magnus' green salt and other impurities in the final product.
A yield of 95% crude $cis\text{-}Pt(NH_3)_2Cl_2$ for Steps 6 and 7 has been obtained. The total yield from Steps 1 through 7 is typically 79%. All platinum can be recovered from discarded materials.

Step 7
Reaction: Purification of crude $cis\text{-}Pt(NH_3)_2Cl_2$

a) First recrystallization
The crude product is added to 0.1 N HCl (about 1 g product to 35 ml 0.1 N HCl), and the solution

4

is warmed to 100°C, filtered hot and then the filtrate is cooled to room temperature for recrystallization.

b) Second recrystallization

Repeat of first recrystallization step.

c) Third recrystallization

Before the third recrystallization step, a test for solubility according to the aforementioned NCI specification should be carried out. If the material does not pass the solubility test an alternative third recrystallization step is carried out as follows: The product of the second recrystallization step is dissolved in DMF. About 50 ml DMF/g of the cis-Pt product is sufficient. After filtration an equivalent volume of 0.1 N HCl is added with stirring and the mixture is permitted to stand with stirring for 20 minutes. The purified cis-Pt product precipitates and after filtration the residue is washed with absolute alcohol and vacuum dried.

It was found that the present product could be retained for a period of 3 days in 0.1 N HCl without any transformation to trans observed by paper chromatography test.

The above method is an improvement over known methods for producing cis-Pt(NH$_3$)$_2$Cl$_2$ in that it gave: (1) unexpectedly high yield of the desired product, (2) a pure cis-isomer free of Magnus' green salt and of the trans-isomer, and (3) a final compound with very low silver content.

Further Examples of the individual steps of the synthesis will now be given.

Example 2

Experiments were run essentially as described in Step 2 above, in which K$_2$PtCl$_6$ is reduced to K$_2$PtCl$_4$ using hydrazine, with the following variations:

| Exp. type | Reagent | Initial temperature | Pt Deposit (Pt°) observed |
|---|---|---|---|
| A-1 | N$_2$H$_4$ . 2HCl | Room temp. | None |
| B-1 | N$_2$H$_4$ | Room temp. | Pt° immediately |
| C-1 | N$_2$H$_4$ . 2HCl | 50°—65°C | Some Pt° on sides of beaker |

Example 3

Experiments were run essentially as described in Step 4 above, in which K$_2$PtI$_4$ is converted to cis-Pt(NH$_3$)$_2$I$_2$ using NH$_4$OH, with the following variations:

| Exp. type | Reagent | Condition | Yield cis-Pt(NH$_3$)$_2$I$_2$ |
|---|---|---|---|
| A-2 | NH$_4$OH | Temp. control gradual to 60°C and gradual additon of NH$_4$OH and control pH not to exceed 7 | 96% |
| B-2 | NH$_4$OH | Stoichiometric NH$_4$OH added to solution at 60°C and without pH control | 77% |

Example 4

Experiments were carried out essentially as described in Step 5 above, in which cis-Pt(NH$_3$)$_2$I$_2$ in aqueous solution is converted with AgNO$_3$ to the cis-[Pt(NH$_3$)$_2$(H$_2$O)$_2$](NO$_3$)$_2$, with the following variations and results:

| Exp. type | Variation addition | Temperature | Yield* crude |
|---|---|---|---|
| A-3 | cis-iodo salt added to AgNO$_3$ | Room temp. | 95% |
| B-3 | cis-iodo salt added to AgNO$_3$ | 50°C | 90% |
| C-3 | cis-iodo salt added to AgNO$_3$ | 90°C | 78% |
| D-3 | AgNO$_3$ added to cis-iodo salt | Room temp. | 84% |

*Since it is difficult to analyze for the diaqua compound, analysis was made for the yield of crude cis-Pt(NH$_3$)$_2$Cl$_2$ (i.e., the product obtained after carrying out step 6 on the filtrates obtained from the tests of this experiment).

5

Example 5

Experiments were carried out essentially as described in Step 6 above, in which cis-[Pt(NH_3)_2(H_2O)_2](NO_3)_2 in solution is treated with KCl to form the crude cis-Pt(NH_3)_2Cl_2, with the following variations and results:

| Exp. type | Conditions | Yield | Observation Magnus' salt |
|---|---|---|---|
| A-4 | 1—Reaction at room temp.<br>2—Warm to 70°C<br>3—Cool to 25°C and filter immediately | 95% | None |
| B-4 | 1—Reaction at room temp.<br>2—Cool to 25°C and filter immediately | 80% | None |
| C-4 | 1—Reaction at room temp.<br>2—Cool to 0°C | — | Layer of green salt |

In another set of experiments three identical solutions containing cis-[Pt(NH_3)_2(H_2O)_2](NO_3)_2 were treated with KCl and the crude cis-platinum complex was permitted to form under the conditions of A-4 and after separation of the precipitate at 25°C, the filtrates were treated, respectively, under the three different conditions noted below, with the following observations:

| Exp. type | Conditions | Observation |
|---|---|---|
| D-4 | Filtrate No. 1 on cold plate<br>Allowed to cool to 15°C<br>Allowed to cool to between 9—10°C | More ppt of cis-Pt formed—no green salt<br>Magnus' green salt came down |
| E-4 | Filtrate No. 2 stood at RT | Traces of Magnus' green salt in 3 hrs. |
| F-4 | Filtrate No. 3 stood at RT | Traces of Magnus' green salt in 3-1/2 hrs. |

The tests showed that Magnus' green salt formed when the temperature fell below about 10°C and when the reaction medium was permitted to stand at room temperature. Even though the Magnus' green salt did not form immediately, the tests show that it is advisable not to let the reaction medium stand at room temperature.

To maximize yield and ensure purity, the cis-Pt(NH_3)_2Cl_2 can be separated from solution in a stepwise fashion. For example, after cooling to room temperature the product is immediately filtered and the solution is permitted to stand until a further precipitate is formed. This is repeated until the formation of a green salt is observed.

Example 6

Experiments were carried out essentially as described in Steps 3 and 4 above in which K_2PtCl_4 is converted to K_2PtI_4 using KI, and then K_2PtI_4 is converted to cis-Pt(NH_3)_2I_2 using a dilute solution of NH_4OH under pH control, with the following variations in pH control in Step 4 and results:

| Exp. type | pH | % Yield[1] | Appearance of product of Step 4 |
|---|---|---|---|
| A-5 | 7 | 91[2] | Dark greenish brown |
| B-5 | 7.5 | 88 | Dark brown |
| C-5 | 8 | 67 | Light brown |

[1]Yield based on Steps 3 and 4.
[2]Average yield based on 7 tests.

Example 7

Samples were prepared using the method of the present invention essentially as described above in Steps 1 through 6 and then purified by recrystallization twice according to the 0.1 N HCl method of Step 7. Samples of the final product were screened according to the aforementioned NCI specification for the chemical NSC-119,875.

A) In the paper chromatography test, no transisomer was detected within the limit of detection (about 1/2%) (14 samples tested).

B) Wet chemical analysis on a typical sample of twice recrystallized product was submitted for analysis of platinum, chlorine and ammonia. The results compared with the NCI specification as follows:

| Elemental component | Spec. % | Analysis % |
|---|---|---|
| Platinum | 65.02±0.30 | 65.2 |
| Chlorine | 23.63±0.30 | 23.5 |
| Hydrogen | 2.02±0.30 | 11.1 (as $NH_3$) |
| Nitrogen | 9.3±0.30 | |

C) The cis-$Pt(NH_3)_2Cl_2$ was observed to be an orange yellow solid which decomposed at 270°C.

D) When a sample was refluxed with thiourea in 0.1 N HCl yellow crystals formed which decomposed at 242°C.

E) IR spectrum run on a Beckman IR-20-X Infrared Spectrometer was compatible with the model spectrum furnished by NCI.

Example 8

Experiments were run essentially as described in Step 1 above, in which $H_2PtCl_6$ is converted to $K_2PtCl_6$, with the following variations:

| Exp. type | Reaction temperature | Yield |
|---|---|---|
| A-7 | 50°C | 95—98% |
| B-7 | RT | 92—93% |

Example 9

A sample of cis-$Pt(NH_3)_2Cl_2$ was prepared using the method of the present invention essentially as described above in Steps 1 through 6 and then purified three times by the recrystallization from 0.1 N HCl. The sample was sent to an independent laboratory for analyses and it was reported that a HPLC (High Performance Liquid Chromatography) recording showed only peaks for cis-$Pt(NH_3)_2Cl_2$ and no other peaks. High sensitivity tracings at the region where small peaks for Magnus' green salt and trans-isomer might appear showed no evidence of such impurities in the product.

Example 10

Samples of crude cis-$Pt(NH_3)_2Cl_2$, prepared using the method of the present invention essentially as described above in Steps 1 through 6 and were analyzed for composition:

1) An IR spectrum run on a Beckman IR-20-X Infrared Spectrometer showed ammine and chloride peaks compatible with the NCI model. An additional unidentified peak was observed at a wavelength of roughly 5.7 $\mu$.

2) In a sample subjected to the NCI paper chromatography test, no trans-isomer was detected within the limit of detection (about 1/2%).

3) Several samples were analyzed for silver content, and analysis showed less than 5 ppm silver.

**Claims**

1. A process which comprises a step in which an aqueous solution of ammonium hydroxide is added to an aqueous dispersion of $M_2PtI_4$ (where M represents sodium or potassium) at a temperature of from 40—60°C to give a cis-$[Pt(NH_3)_2I_2]$ complex in such a manner that the pH does not exceed 7.5.

2. A process as claimed in claim 1, wherein the said step is carried out under a controlled, gradually rising temperature profile.

3. A process as claimed in claim 2, wherein the temperature is increased stepwise.

4. A process as claimed in claim 1, wherein the $M_2PtI_4$ complex is dispersed in water and heated to about 40°C, a dilute solution of ammonium hydroxide is added in such a manner that the pH does not exceed 7, the reaction mixture is then heated to about 60°C, additional ammonium hydroxide is added slowly in an amount sufficient to raise the pH to about 7, and the resultant mixture is then cooled to room temperature thereby precipitating cis-$[Pt(NH_3)_2I_2]$.

5. A process as claimed in any one of claims 1 to 4, which further comprises treating the cis-

$[Pt(NH_3)_2I_2]$with an aqueous solution of $AgNO_3$ to form a solution containing cis-$[Pt(NH_3)_2(H_2O)_2](NO_3)_2$ and a silver iodide precipitate, which are then separated.

6. A process as claimed in claim 5, wherein washed and dried cis-$Pt(NH_3)_2I_2$ is added with vigorous stirring to the aqueous solution of $AgNO_3$ at room temperature, the mixture is warmed to a temperature of about 50°C for a period of time sufficient to substantially complete the reaction, then the reaction mixture is cooled to room temperature.

7. A process as claimed in claim 5 or in claim 6, wherein the amount of $AgNO_3$ used is slightly less than the stoichiometric amount needed for complete conversion of cis-$[Pt(NH_3)_2I_2]$ to cis-$[Pt(NH_3)_2(H_2O)_2](NO_3)_2$ and, after separation of AgI, a small but effective amount of solid NaCl or KCl is added to the solution of cis-$[Pt(NH_3)_2(H_2O)_2](NO_3)_2$ to precipitate remaining $Ag^+$ ions, and precipitated AgCl is separated from the solution.

8. A process as claimed in claim 5 or in claim 6, wherein the concentration of $Ag^+$ in the reaction mixture is monitored with a silver/silver chloride electrode to minimise the excess $Ag^+$ in solution after the reaction has proceeded to completion.

9. A process as claimed in any one of claims 5 to 8, which further comprises converting the solution containing cis-$[Pt(NH_3)_2(H_2O)_2](NO_3)_2$, by adding NaCl or KCl rapidly to the solution at room temperature, raising the temperature to a temperature not exceeding 75°C, maintaining the elevated temperature for sufficient time to complete the formation of cis-$[Pt(NH_3)_2Cl_2]$, cooling the reaction mixture and separating the cis-$[Pt(NH_3)_2Cl_2]$ precipitate formed.

10. A process as claimed in claim 9, wherein the reaction mixture containing cis-$[Pt(NH_3)_2Cl_2]$ is cooled to not less than 10°C and the precipitate is separated before the appearance of Magnus' green salt.

11. A process as claimed in claim 9 or claim 10, wherein the reaction mixture is cooled to room temperature.

12. A process as claimed in any one of claims 9 to 11, wherein the cis-$[Pt(NH_3)_2Cl_2]$ is purified by a method comprising heating cis-$[Pt(NH_3)_2Cl_2]$ in dilute HCl to about 100°C, filtering the solution while still hot and cooling the filtrate to precipitate cis-$[Pt(NH_3)_2Cl_2]$.

13. A process as claimed in any one of claims 1 to 12, wherein the said $M_2PtI_4$ has been obtained by admixing an aqueous solution of KI and an aqueous solution of $M_2PtCl_4$ (where M represents sodium or potassium) at a temperature not in excess of 85°C.

14. A process as claimed in any one of claims 1 to 12, wherein the said $M_2PtI_4$ has been obtained by adding an aqueous solution of hydrazine dihydrochloride to an aqueous dispersion of $M_2PtCl_6$ (where M represents sodium or potassium) at room temperature, raising the temperature stepwise to a temperature not greater than 85°C to form $M_2PtCl_4$ and converting the $M_2PtCl_4$ to the said $M_2PtI_4$.

15. A process as claimed in any one of claims 1 to 14, wherein the said $M_2PtI_4$ has been obtained by adding an aqueous solution of MCl (where M represents sodium or potassium) to an aqueous solution of $H_2PtCl_6$ at a temperature in the range of from room temperature to 60°C, cooling the reaction to below room temperature to precipitate $M_2PtCl_6$, which is separated and converted to $M_2PtCl_4$ which in turn is converted to the said $M_2PtI_4$.

## Revendications

1. Procédé qui comprend un stade opératoire dans lequel on ajoute une solution aqueuse d'hydroxyde d'ammonium à une dispersion aqueuse de $M_2PtI_4$ (M représentant le sodium ou le potassium) à une température de 40 à 60°C, afin de former un complexe de cis-$[Pt(NH_3)_2I_2]$ dans des conditions telles que le pH ne dépasse pas 7,5.

2. Procédé selon la revendication 1, dans lequel ledit stade opératoire est réalisé avec profil de température contrôlé, montant progressivement.

3. Procédé selon la revendication 2, dans lequel la température est augmentée par paliers.

4. Procédé selon la revendication 1, dans lequel on disperse le complexe $M_2PtI_4$ dans l'eau et on chauffe à 40°C environ, on ajoute une solution diluée d'hydroxyde d'ammonium dans des conditions telles que le pH ne dépasse pas 7, on chauffe ensuite le mélange de réaction à 60°C environ, on ajoute lentement des compléments d'hydroxyde d'ammonium en quantité suffisante pour porter le pH à 7 environ puis on refroidit le mélange à température ambiante, provoquant ainsi la précipitation du cis-$[Pt(NH_3)_2I_2]$.

5. Procédé selon l'une quelconque des revendications 1 à 4, qui comprend en outre le traitement du cis-$[Pt(NH_3)_2I_2]$ par une solution aqueuse d'$AgNO_3$, afin de former une solution contenant le cis-$[Pt(NH_3)_2(H_2O)_2](NO_3)_2$ et un précipité d'iodure d'argent qu'on sépare ensuite.

6. Procédé selon la revendication 5, dans lequel on ajoute le cis-$Pt(NH_3)_2I_2$ lavé et séché sous agitation vigoureuse à la solution aqueuse d'$AgNO_3$ à température ambiante, on chauffe le mélange à une température d'environ 50°C pendant une durée suffisante pour achever pratiquement la réaction puis on refroidit le mélange de réaction à température ambiante.

7. Procédé selon la revendication 5 ou 6, dans lequel la quantité d'$AgNO_3$ utilisée est légèrement inférieure à la quantité théorique nécessaire pour une conversion complère du cis-$[Pt(NH_3)_2I_2]$ en cis-$[Pt(NH_3)_2(H_2O)_2](NO_3)_2$ et, après séparation d'AgI, on ajoute une proportion faible mais efficace de NaCl

ou KCl solide à la solution de cis-$[Pt(NH_3)_2(H_2O)_2](NO_3)_2$ pour précipiter les ions $Ag^+$ restants et on sépare l'AgCl qui a précipité de la solution.

8. Procédé selon la revendication 5 ou 6, dans lequel la concentration d'$Ag^+$ dans le mélange de réaction est suivie à l'aide d'une électrode à l'argent/chlorure d'argent afin de réduire au minimum l'excès d'$Ag^+$ dans la solution lorsque la réaction est terminée.

9. Procédé selon l'une quelconque des revendications 5 à 8, qui comprend en outre la conversion de la solution contenant le cis-$[Pt(NH_3)_2(H_2O)](NO_3)_2$ par addition rapide de NaCl ou KCl à la solution à température ambiante, augmentation de la température jusqu'à un niveau ne dépassant pas 75°C, le maintien de la température élevée pendant une durée suffisante pour compléter la formation du cis-$[Pt(NH_3)_2Cl_2]$, le refroidissement du mélange de réaction et la séparation du précipité de cis-$[Pt(NH_3)_2Cl_2]$ formé.

10. Procédé selon la revendication 9, dans lequel le mélange de réaction contenant le cis-$[Pt(NH_3)_2Cl_2]$ est refroidi à une température non inférieure à 10°C et le précipité séparé avant l'apparition du sel vert de Magnus.

11. Procédé selon la revendication 9 ou 10, dans lequel le mélange de réaction est refroidi à température ambiante.

12. Procédé selon l'une quelconque des revendications 9 à 11, dans lequel le cis-$[Pt(NH_3)_2Cl_2]$ est purifié par un mode opératoire qui comprend le chauffage du cis-$[Pt(NH_3)_2Cl_2]$ dans HCl dilué à une température d'environ 100°C, la filtration de la solution encore chaude et le refroidissement du filtrat afin de faire précipiter le cis-$[Pt(NH_3)_2Cl_2]$.

13. Procédé selon l'un quelconque des revendications 1 à 12, dans lequel ledit $M_2PtI_4$ a été obtenu par mélange d'une solution aqueuse de KI et d'un solution aqueuse de $M_2PtCl_4$ (M représentant le sodium ou le potassium) à une température ne dépassant pas 85°C.

14. Procédé selon l'une quelconque des revendications 1 à 12, dans lequel ledit $M_2PtI_4$ a été obtenu par addition d'une solution aqueuse de dichlorhydrate d'hydrazine à une dispersion aqueuse de $M_2PtCl_6$ (M représentant le sodium ou le potassium) à température ambiante, augmentation de la température par paliers jusqu'à un niveau ne dépassant pas 85°C, avec formation de $M_2PtCl_4$, et conversion du $M_2PtCl_4$ en ledit $M_2PtI_4$.

15. Procédé selon l'une quelconque des revendications 1 à 14, dans lequel ledit $M_2PtI_4$ a été obtenu par addition d'une solution aqueuse de MCl (M représentant le sodium ou le potassium) à une solution aqueuse de $H_2PtCl_6$ à une température allant de la température ambiante jusqu'à 60°C, refroidissement du mélange de réaction à une température inférieure à la température ambiante, provoquant la précipitation de $M_2PtCl_6$ qu'on sépare et qu'on convertit en $M_2PtCl_4$ lui-même converti à son tour en ledit $M_2PtI_4$.

## Patentansprüche

1. Verfahren, welches einen Schritt umfaßt, in welchem eine wässerige Lösung von Ammoniumhydroxid zu einer wässerigen Dispersion von $M_2PtI_4$ bei einer Temperatur zwischen 40 und 60°C hinzugefügt wird (wobei M Natrium oder Kalium bedeutet), um einen cis-$[Pt(NH_3)_2I_2]$ Komplex zu erhalten, wobei der pH-Wert nicht 7.5 übersteigt.

2. Verfahren nach Anspruch 1, dadurch gekennzeichnet, daß der erwähnte Schritt unter einem kontrollierten, allmählich ansteigenden Temperaturprofil ausgeführt wird.

3. Verfahren nach Anspruch 2, dadurch gekennzeichnet, daß die Temperatur schrittweise angehoben wird.

4. Verfahren nach Anspruch 1, dadurch gekennzeichnet, daß der $M_2PtI_4$-Komplex in Wasser dispergiert und bis etwa 40°C erhitzt wird, daß eine verdünnte Lösung von Ammoniumhydroxid in solcher Weise hinzugeführt wird, daß der pH-Wert 7 nicht übersteigt, daß die Reaktionsmischung dann auf etwa 60°C erhitzt wird, daß zusätzliches Ammoniumhydroxid langsam zugefügt wird, in einem Ausmaß, das ausreicht, um den pH-Wert auf etwa 7 zu erhöhen und daß die resultierende Mischung dann auf Raumtemperatur abgekühlt wird, wobei cis-$[Pt(NH_3)_2I_2]$ ausfällt.

5. Verfahren nach einem der Ansprüche 1 bis 4, welches weiter umfaßt die Behandlung des cis-$[Pt(NH_3)_2I_2]$ mit einer wässrigen Lösung von $AgNO_3$, um eine Lösung zu bilden, welche cis-$[Pt(NH_3)_2(H_2O)_2](NO_3)_2$ enthält und einen Silberiodidniederschlag, welche dann voneinander getrennt werden.

6. Verfahren nach Anspruch 5, in welchem gewaschenes und getrocknetes cis-$Pt(NH_3)_2I_2$ unter heftigem Rühren zur zur wässrigen Lösung von $AgNO_3$ bei Zimmertemperatur hinzugefügt wird, die Mischung auf eine Temperatur von etwa 50°C für eine Dauer erwärmt wird, welche ausreicht, um im wesentlichen die Reaktion zu vollenden und daß dann die Reaktionsmischung auf Zimmertemperatur abgekühlt wird.

7. Verfahren nach Anspruch 5 oder 6, wobei die verwendete Menge von $AgNO_3$ etwas geringer ist als die stöchiometrische Menge, die für eine Vollständige Umwandlung von cis-$[Pt(NH_3)_2I_2]$ zur cis-$[Pt(NH_3)_2(H_2O)_2](NO_3)_2$ notwendig wäre, und daß nach der Trennung von AgI eine kleine aber wirksame Menge von festem NaCl oder KCl zur Lösung von cis-$[Pt(NH_3)_2(H_2O)_2](NO_3)_2$ hinzugefügt wird, um die

verbleibenden AG$^+$-Ionen niederzuschlagen und daß dann das niedergeschlagene AgCl von der Lösung getrennt wird.

8. Verfahren nach Anspruch 5 oder 6, wobei die Konzentration von Ag$^+$ in der Reaktionsmischung mit einer Silber/Silberchloridelektrode überwacht wird, um den Überschuß an Ag$^+$ in der Lösung nach Vollendung der Reaktion so gering wie möglich zu halten.

9. Verfahren nach einem der Ansprüche 5 bis 8, welches weiter die Umwandlung der Lösung, welche cis-[Pt(NH$_3$)$_2$(H$_2$O)$_2$](NO$_3$)$_2$ enthält, durch schnelle Zufügung von NaCl oder KCl zur Lösung bei Zimmertemperatur umfaßt, worauf die Temperatur auf einen Wert, der 75°C nicht übersteigt, erhöht wird und die erhöhte Temperatur für hinreichende Zeit aufrechterhalten wird, um die Bildung von cis-[Pt(NH$_3$)$_2$Cl$_2$] zu vollende, woraufhin die Reaktionsmischung abgekühlt und der gebildete cis-[Pt(NH$_3$)$_2$Cl$_2$]-Niederschlag abgetrennt wird.

10. Verfahren nach Anspruch 9, wobei die Reaktionsmischung, welche cis-[Pt(NH$_3$)$_2$Cl$_2$] enthält, auf nicht wenigen als 10°C abgekühlt wird und der Niederschlag abgetrennt wird, bevor grünes Magnussalz erscheint.

11. Verfahren nach Anspruch 9 oder 10, wobei die Reaktionsmischung auf Zimmertemperatur abkühlt wird.

12. Verfahren nach einem der Ansprüche 9 bis 11, wobei das cis-[Pt(NH$_3$)$_2$Cl$_2$] nach einem Verfahren gereinigt wird, welches die Erhitzung des cis-[Pt(NH$_3$)$_2$Cl$_2$] in verdünnter Salzsäure bis etwa 100°C, das Filtern der heißen Lösung und Abkühlung des Filtrats zur Ausfällung von cis-[Pt(NH$_3$)$_2$Cl$_2$] umfaßt.

13. Verfahren nach einem der Ansprüche 1 bis 12, wobei das erwähnte M$_2$PtI$_4$ erhalten wurde, indem eine wässerige Lösung von KI und eine wässerige Lösung von M$_2$PtCl$_4$ (wobei M Natrium oder Kalium bedeutet) bei einer Temperatur nicht overhalb 85°C zusammengemischt wurden.

14. Verfahren nach einem der Ansprüche 1 bis 12, wobei das erwähnte M$_2$PtI$_4$ erhalten wurde, indem eine wässerige Lösung von Hydrazindihydrochlorid einer wässerigen Dispersion von M$_2$PtCl$_6$ (wobei M Natrium oder Kalium bedeutet) bei Zimmertemperatur zugefügt, die Temperatur schrittweise auf nicht mehr als 85°C erhöht wurde, um M$_2$PtCl$_4$ zu bilden und das M$_2$PtCl$_4$ zu dem erwähnten M$_2$PtI$_4$ umgewandelt wurde.

15. Verfahren nach einem der Ansprüche 1 bis 14, wobei das erwähnte M$_2$PtI$_4$ erhalten wurde, indem eine wässerige Lösung von MCl (wobei M Natrium oder Kalium bedeutet) einer wässerigen Lösung von H$_2$PtCl$_6$ bei einer Temperatur zwischen Zimmertemperatur und 60°C zugefügt wurde, die Reaktion unter Zimmertemperatur abgekühlt wurde, um M$_2$PtCl$_6$ auszufällen, welches abgetrennt und zu M$_2$PtCl$_4$ umgewandelt wurde, welches seinerseits in M$_2$PtI$_4$ umgewandelt wurde.